# EUROPEAN PATENT APPLICATION

(11) **EP 1 632 211 A1**
(43) Date of publication of application: **08.03.2006**
(21) Application number: 04021284.7
(22) Date of filing: 07.09.2004
(51) Int. Cl.: A61K 6/083, A61L 15/12, C08L 33/02

(54) **Dental resin-modified polyalkenoate cement composition**

(71) Applicant: S & C Polymer Silicon- und Composite-Spezialitäten GmbH, 25335 Elmshorn (DE)
(72) Inventor: Engelbrecht, Jürgen c/o S&C Polymer Spezialitäten, 25335 Elmshorn (DE); Akinmade, Ademola Olaseni, 25335 Elmshorn (DE)
(74) Representative: Forstmeyer, Dietmar

(57) **Abstract**

The dental resin-modified polyalkenoate cement composition according to the present invention especially contains substantially conjugated hydrocarbons that act as a radiant-energy or heat-energy absorbing substance. Upon irradiation, the composition improves the clinical performance of a wide range of dental polyalkenoate cements products such as: sealants; orthodontic and other adhesive cement and general adhesive restoratives, especially in situations of low saliva management, e.g. in paedriatic and geriatric usage.

## Description

The present invention relates to a dental resin-modified polyalkenoate cement composition which contains at least one substantially conjugated hydrocarbon that acts as a radiant-energy or heat-energy absorbing substance.

The dental polyalkenoate class of cements includes the commercial glass-ionomer and the zinc polycarboxylate cements. The dental resin-modified glass-ionomer cement [US Patent No. 4,872,936 Oct 1989] is a variant of the dental glass-ionomer cement, invented to tackle some of the limitations of the older "Classical" variant [US Patent No. 3814717]. The potential of the Classical glass-ionomer cement, based on its attractive features, is still to be fully realized. It is particularly biocompatible and exhibits good chemical adhesion to tooth structure. Classical glass-ionomer cement and to some extent the resin-modified variant are susceptible to saliva attack when freshly mixed. In this deleterious process, the water content of the cement increases and its metal ion-based cross-linking setting decreases. This results in more opaque restorations with lower surface hardness and, as a consequence, worse clinical wear and performance.

The dental resin-modified glass-ionomer cement comprises, in addition to the constituents of the Classical variant, polymerisable unsaturated monomers and/or oligomers and/or prepolymers containing acid groups and/or their reactive acid-derivatives. The constituents of the Classical dental glass-ionomer cement are finely ground fluoro- aluminosilicate glass powder, polyalkenoic acid(s) and water. There exist other dental resin-modified polyalkenoate cements, based not on glasses but on basic oxides [European Patent No. EP0883586].

In this way, the resin-modified polyalkenoate cements undergo both the water-based acid-base "ionomer" reaction and the polymerisation of monomers and other polymerisable groups by the action of external light and/or redox-based free radicals. In general the inclusion of resins into dental formulations has improved their aesthetics, rapid development of strength, hardness, bond strengths and other mechanical properties and wear. On the other hand, these resin-based products are less hydrophilic and require more attention to achieve adequate (albeit higher) bond strengths. They also wet less readily into fissures than Classical glass-ionomer cements. By choice of hydrophilicity of resin and the quantity in the cement composition, the balance between "resin" and water-based "ionomer" can be varied and indeed does differ amongst the commercial resin-modified glass-ionomer cements. Therefore, commercial resin-modified glass-ionomer cements exhibit varying degrees of the positive and negative features associated with each of their components: i) resin composites; and ii) hydrophilic ionomer.

It is always desirable to have other "tools" with which to design cements, in the case of the resin-modified glass-ionomer cements, in order to have more/other combinations of the features of the constituent resin and ionomer parts of these materials.

The object of this invention is to present resin-modified polyalkenoate cements with an improved hardness, bond (cohesive) strength; susceptibility to early water attack and solubility.

This object is solved by a cement as disclosed in the claims. The inventive composition comprises at least one substantially conjugated hydrocarbon compound which absorbs incident radiant energy and thereby makes the cement set faster to the benefit of cement time-dependent properties.

When radiant energy is shone on an inventive cement its temperature rises to the benefit of relatively disadvantageous non and low resin-content polyalkenoate cement features. These include: slow speed of set; susceptibility to early water solubility; sluggish attainment of hardness; sluggish attainment of bond strength (cohesive strength), etc. Therefore, the above-mentioned substantially conjugated hydrocarbon-containing cements, upon radiant energy irradiation, improve the clinical performance of a wide range of dental polyalkenoate cements products. The formulations can be used as: sealants; orthodontic and other adhesive cements; and general adhesive restoratives, especially in situations of low saliva management, e.g. in paedriatic and geriatric usage.

Examples of such radiant energy or heat energy absorbing substantially conjugated hydrocarbons are multiple benzene structures, conjugated hydrocarbon chains, and combinations thereof such as aromatic hydrocarbons, multiple double-bond hydrocarbon chains, chain-aromatic mixtures, reacted combinations thereof, and equivalents. These substances have simple hydrogen, hydroxyl, or carboxylic groups attached to the structures and act as energy-absorbing substances, absorbing portions of the electromagnetic spectrum. Other multiple benzene structures can also be as simple as naptha-based structures or anthracene-based structures. Useful substantially conjugated hydrocarbons that are benzene structures that are radiant energy absorbing include 9,10-bis(phenylethynyl)-anthracene, perylene, naphtho[2,3-a] pyrene, trans-4,4'-diphenylstilbene, 9,10-diphenylanthracene,5,12-bis(phenylethynyl)-napthacene, coronene, fluoranthene, and equivalents. The known substantially conjugated hydrocarbon chains include caroteneoids, such as bixin, lycoxanthin, lycophil, canthaxanthin, capsanthin, cryptoxanthin, isomers of carotene, and lycopene. Of the known substantially conjugated hydrocarbons, 9,10-bis (phenylethynyl)-anthracene, perylene, and isomers of carotene are preferred. Of the known substantially conjugated hydrocarbons, carboxyl-substituted hydrocarbons are also preferred. Other examples of radiant energy absorbers are aromatic compounds such as 7-diethylamino 4-methyl coumarin, henna, and alizarin which are less stable compared to the above-defined substantially conjugated hydrocarbons. Henna, a red dye known for at least 4,000 years, is a double-ketonated naphtha hydroxide. Alizarin, a red dye, is a double-ketonated anthracene meta double hydroxide. The preferred substantially conjugated hydrocarbon of this invention is β-carotene and vitamin A and their derivatives or combinations of two or more of such compounds.

In particular, the dental polyalkenoate cement compositions according to the present invention preferably have a content of the substantially conjugated hydrocarbons in the amount from 0.001 to 10 % by weight based on the cement composition, but preferable from 0.01 - 1% by weight.

In this invention, the term "radiant energy" includes electromagnetic radiation (from any energy source) in the region of microwave, infrared, visible and ultraviolet regions of the electromagnetic spectrum. Preferable is the use of light having a wavelength from 300 to 800 nm, more preferable from 320 to 780 nm, as widely used in the current dental remedy.

The temperature of the cement composition according to the present invention increases upon irradiation with light, and the time of initial setting is accelerated, whereby the susceptibility to attack by external water becomes less.

The dental cement composition of the present inventon can also comprise a fluoro-aluminosilicate glass or basic oxides thereof. These compounds are preferably used as a powder which - in a preferred embodiment - can be finally ground.

As the fluoro aluminosilicate glasses fluoro aluminosilicate glass powders and the like, which are generally used for dental glass-ionomer cements, are preferred. Of these, such fluoro aluminosilicate glass powders are preferred which have as the main components from 10 to 25 % by weight of A1, from 5 to 30 % by weight of Si, from 1 to 30 % by weight of F, from 0 to 20 % by weight of Sr, from 0 to 20 % by weight of Ca, and from 0 to 10 % by weight of alkali metal ions (e.g., Na, K etc.), based on the whole weight of the glass. Such glasses can be prepared by mixing raw materials containing these components and melting the mixture, and then cooling and pulverizing so as to have a mean particle size of from about 0.02 to 20 pm.

Of the basic oxide variant of the water-based polyalkenoate cement according to the present invention, preferable is the zinc oxide and deactivated zinc oxide/magnesium oxides of the zinc polycarboxylate cements. Preferred also are the newer zinc-based dental compositions [cf. European Patent No. EP0883586, which is incorporated herein by reference]. Other less preferred basic oxide cements include those prepared from Be, Zn, Cu, Mg, Ca, Sr and Ba and combinations thereof.

Resins, which can be added to the dental cement compositions are known per se. In this invention, "resin" are preferably defined as polymerisable unsaturated mono- or di- or tri-methacrylate monomers and/or oligomers and/or pre-polymers containing acid groups and/or their reactive acid-derivatives in powder, liquid or bead form. The cement formulation could also include known polymerization activators, initiators and other redox reaction systems. In this invention, resin-modified polyalkenoate cements and "resin" are defined in accordance with the US Patent No. 4,872,936 which is incorporated herein by reference, especially concerning this disclosure.

Furthermore polyalkenoic acids and their use in dental cements are known per se. They are for example, described in Werner Kullmann, Atlas der Zahnerhaltung, Hanser Verlag, München (1990). The disclosure of this Atlas, and especially its disclosure concerning polyalkenoic acids is herewith enclosed by reference.

Unforeseen it could be observed that due to the content of energy absorbing substances the resin content of the cement could be lower than in commercial cements still achieving similar performance. Surprisingly it could be observed, that also formulations with a resin content of 20%, and more surprisingly formulations with a resin content of 10%, and even 5% are still achieving these performances.

The substantially conjugated hydrocarbons may be contained in any of the respective components of the cement: in the fluoro-aluminosilicate glass powder; in the aqueous solution of polyalkenoic acid(s); or in the components constituting the dental glass-ionomer cement composition in a powder, liquid or paste format. In addition to the resin-modified dental polyalkenoate cement compositions according to the present invention usual additives can be added like rheology modifiers, light absorbers like known ultraviolet light absorbers, plasticizers, antioxidants, bactericides, surfactants, etc., if desired.

The dental resin-modified polyalkenoate cement compositions according to the present invention will be hereunder described in more detail with reference to the following glass-ionomer Examples, but it is to be construed that the present invention should not be limited thereto. In the examples β-carotene is used as a conjugated energy-absorbing hydrocarbon.

Example 1. The Effect of Resin and substantially conjugated hydrocarbon inclusion on the temperature rise and setting properties of Glass-ionomer Cements

| **Liquid Constituents** | **Weight % of Liquid Constituents** | | | | |
|---|---|---|---|---|---|
| | Liquid-1 | Liquid-2 | Liquid-3 | Liquid-4 | Liquid-5 |
| β-carotene | 0.08 | 0.08 | 0.08 | 0.08 | 0.0 |
| RM Lute liquid | 0 | 3.3 | 6.6 | 12.5 | 12.5 |
| Vitro Molar Liquid | 99.9 | 96.7 | 93.4 | 87.5 | 87.5 |
| | 100 | 100 | 100 | 100 | 100 |
| Temperature rise °C | 4.0 | 4.6 | 9.8 | 11.4 | 0.6 |
| Setting Time without light activation [sec] | 240 | 240 | 240 | 240 | 240 |
| Setting Time with 40s radiant energy irradiation [sec] | 215 | 195 | 160 | 128 | 240 |
| Decrease in Setting Time with 40s energy irradiation [sec] | 25 | 45 | 80 | 112 | 0 |
| Decrease in Setting Time with 40s energy irradiation [%] | 10.4 | 18.8 | 33.3 | 46.7 | 0.0 |

| | | | | | |
|---|---|---|---|---|---|
| *RM Lute is a non light-cure resin-modified glass-ionomer commercial product of Strrouf Pharmaceuticals India. The cements were formed by mixing Vitro Molar powder with the liquids at powder: liquid ratio of 1,4:1. The radiant energy source employed in all of the examples presented was Power Pac manufactured by American Medical Technologies (AMT), Corpus Cristi, USA. | | | | | |

It can be concluded:
1. β-Carotene has the effect of significantly raising the temperature of the glass-ionomer cements.
2. In the absence of β-Carotene classical and resin-modified glass-ionomer cements have very low temperature rise when irradiated with our energy source.
3. The inclusion of β-Carotene and especially in conjunction with resins has the effect of creating novel glass-ionomer cements with command-cure properties similar in some respect to those of traditional commercial resin-modified glass-ionomer cements.

Example 1 has outlined the temperature rise feature and the benefit this has in enabling cements to set faster. Other potential benefits of these β-Carotene -containing products and the proposed curing method have been evaluated and are described following:

Example 2. Increase in Early Surface Hardness - of potential benefit for fissure sealing applications and as an alternative to the varnishing of dental glass-ionomer restorations.

| **Time interval** | **Shore-D Hardness*** | |
|---|---|---|
| | **Without energy irradiation** | **With 40 s energy irradiation** |
| 5 mins from start of mix | 0 | 4 |
| 10 mins from start of mix | 20 | 29 |
| 25 mins from start of mix | 50 | 63 |
| 60 mins from start of mix | 80 | 81 |

The cement of this experiment was Liquid-3 mixed with the Vitro Molar powder, mixed at powder: liquid ratio of 1,4:1 and irradiated with radiant energy for 40s.

Example 3. Development of early high bond strength to un-etched enamel - of potential benefit in the bonding of orthodontic and other dental appliances

| **Time Interval** | **Shear Bond Strength** | |
|---|---|---|
| | **Without light irradiation** | **With 40 s energy irradiation** |
| 30mins ± 5 mins | 0 MPa | 2.1 MPa |
| 3 hours ± 15 mins | 2.2 MPa | 6.5 MPa |

Liquid 3 was mixed with Vitro Molar powder at powder: liquid of 1.4:1 and applied to poly(acrylic acid) conditioned enamel and irradiated with radiant energy for 40s.

## Claims

1. A dental resin-modified polyalkenoate cement composition comprising at least one substantially conjugated hydrocarbon compound which is able to absorb radiant energy within the range of the microwave, infrared, visible and/or ultraviolet spectrum, and to transform this energy into heat.

2. The dental cement composition according to Claim 1 wherein the radiant energy has a wavelength from 300 - 800 nm.

3. The dental cement composition according to Claims 1 or 2 wherein the substantially conjugated hydrocarbon compound is a caroteneoid.

4. The dental cement composition according to any one of Claims 1 - 3 wherein the substantially conjugated hydrocarbon compound is β-carotene.

5. The dental cement composition according to any one of Claims 1-4 wherein the substantially conjugated hydrocarbons are contained, based on the whole composition, in an amount from 0,001% to 10% by weight and preferably from 0,01% to 1% by weight.

6. The dental cement composition according to any one of Claims 1 - 5 wherein the resin is present as a bead, powder or liquid, or in a combination of these forms.

7. The dental cement composition according to any one of claims 1 - 6, wherein the resin is included in any one of the components of the glass-ionomer powder, liquid or paste.

8. The dental cement composition according to any one of claims 1 - 7, wherein the resin is a mono- or di- or tri- or multi-methacrylate and optionally includes polymerization activators, initiators and/or other redox reaction systems.

9. The dental cement composition according to any one of Claims 1-7, additionally containing vitamin A and/or its derivatives.

10. The dental cement composition according to any one of Claims 1-9, wherein the resin content is lower than 20 weight percent, based on the whole composition.

11. The dental cement composition according to any one of Claims 1-10 where the resin content is lower than 10 weight percent, based on the whole composition.

12. The dental cement composition according to any one of Claims 1-11 wherein the resin content is lower than 5 weight percent, based on the whole composition.

13. The dental cement composition according to any one of Claims 1-11 wherein the basic oxide component of the acid-base cement is an acid-susceptible glass or basic oxide(s) or combinations thereof.
